# EUROPEAN PATENT APPLICATION

(11) **EP 4 268 706 A1**
(43) Date of publication of application: **01.11.2023**
(21) Application number: 21909599.9
(22) Date of filing: 27.12.2021
(51) Int. Cl.: A61B 5/00, G16H 80/00

(54) **MEDICAL INFORMATION DISPLAY SYSTEM AND MEDICAL SYSTEM**

(30) Priority: 25.12.2020 CN 202011565773; 25.12.2020 CN 202011562887
(71) Applicant: Shenzhen Mindray Bio-Medical Electronics Co., Ltd, Shenzhen, Guangdong 518057 (CN)
(72) Inventor: WANG, Shengping, Shenzhen, Guangdong 518057 (CN); DAI, Weiwei, Shenzhen, Guangdong 518057 (CN); WANG, Cheng, Shenzhen, Guangdong 518057 (CN); LIANG, Liming, Shenzhen, Guangdong 518057 (CN); ZHU, Jianguang, Shenzhen, Guangdong 518057 (CN); LIU, Shuo, Shenzhen, Guangdong 518057 (CN)
(74) Representative: KIPA AB
(86) International application number: PCT/CN2021/141727
(87) International publication number: WO 2022/135604

(57) **Abstract**

A medical information display system (100) and a medical system. The medical information display system (100) comprises: a communication interface (104), a storage device (103), a display device (102), and one or more processors (101). The processors (101) are configured to execute program instructions stored in the storage device (103), thus allowing the processors (101) to execute the following steps: acquiring via the communication interface (104) vital signs information collected by a monitoring device (520) and ultrasonic information generated by an ultrasonic device (510), controlling the storage device (103) to store the vital signs information and the ultrasonic information according to timestamps; when a selection instruction with respect to one display area of multiple display areas of a display and observation interface (210) is acquired, controlling the display device (102) to display a detail interface of a target object of interest corresponding to the display area being selected; and when a review instruction with respect to a review hotkey is acquired, controlling the display device (102) to display in the detail interface a review interface of the target object of interest, the review interface being used for displaying review information of the target object of interest, and the review interface comprising the vital signs information and the ultrasonic information.

## Description

### TECHNICAL FIELD

The disclosure generally relates to a technical field of medical device, and more specifically to a medical information display system and a medical system.

### BACKGROUND

Diagnosis of doctor on patient condition requires comprehensive analysis of various aspects of information, such as physiological parameters monitored by various instruments, ultrasonic examination images, and complexion and expression of patient, and so on. However, such information is scattered across various device terminals, making it difficult for the doctor to easily view, compare and analyze uniformly. Moreover, when diagnosing the patient condition, doctor sometimes not only needs to know the current patient situation, but also needs to understand the trend of changes in the patient condition over a period of time. Therefore, it is necessary to review and view the historical medical data information of the patient, but this information is either not stored or stored separately in various terminals or systems, which cannot be conveniently reviewed and compared for analysis.

### SUMMARY

The present disclosure is provided, aiming at at least one of the above defects.

Specifically, a first aspect of this disclosure provides a medical information display system, including:
a communication interface, which is configured to communicatively connect with at least one monitoring device and/or at least one ultrasonic device, wherein each monitoring device is configured to acquire vital sign information of a target object which is monitored by the monitoring device, and each ultrasonic device is configured to perform an ultrasonic examination on a target object and generate ultrasonic information of said target object;
a storage device, which is configured to store executable program instructions, as well as to store the vital sign information which is acquired by the monitoring device and the ultrasonic information which is generated by the ultrasonic device;
a display device, which is configured to display the vital sign information and the ultrasonic information of one or more target objects;
one or more processors, which are configured to execute the program instructions which are stored in the storage device to enable the processor(s) to perform following steps:
   obtaining the vital sign information which is acquired by the monitoring device and the ultrasonic information which is generated by the ultrasonic device through the communication interface;
   controlling the storage device to store the vital sign information and the ultrasonic information according to a timestamp;
   controlling the display device to provide a display observation interface, which includes a plurality of display areas, each display area correspondingly displays at least part of the vital sign information of one target object;
   when obtaining a selection instruction for one display area of the plurality of display areas, controlling the display device to display a detail interface of an interested target object, which corresponds to the selected display area, wherein the detail interface is set with a review hotkey; and
   when obtaining a review instruction for the review hotkey, controlling the display device to display a review interface for the interested target object on the detail interface, wherein the review interface is configured to display review information of the interested target object, and the review information includes the vital sign information and the ultrasonic information of the interested target object; wherein, the ultrasonic information is displayed with the timestamp, or the vital sign information and the ultrasonic information are correlatively displayed based on the timestamp.

Specifically, a second aspect of this disclosure provides a medical information display system, which includes:
a communication interface, which is configured to communicatively connect with at least one of a monitoring device, an examination device, a treatment device, and a camera device, wherein the monitoring device is configured to acquire vital sign information of a target object which is monitored by the monitoring device, the examination device is configured to exam the target object and generate examination information of the target object, the treatment device is configured to treat the target object and output treatment information, the camera device is configured to acquire imaging information of the target object;
a storage device, which is configured to store executable program instructions, as well as to store medical information of the target object, wherein the medical information includes at least two types of the vital sign information, the examination information, the treatment information, and the imaging information;
one or more processors, which are configured to execute the program instructions which are stored in the storage device to enable the processor(s) to perform following steps: obtaining the medical information of a plurality of target objects through the communication interface; wherein the medical information of each target object includes at least two types of the vital sign information, the examination information, the treatment information, and the imaging information; controlling the storage device to store the obtained medical information; and controlling the display device to display the medical information of the plurality of target objects; and controlling, when a selection instruction for one target object is received, the display device to display detailed information of the medical information of a selected target object; and
a display device, which is configured to display the medical information.

Specifically, a third aspect of this disclosure provides a medical system, which includes:
at least one monitoring device, wherein each monitoring device is configured to acquire vital sign information of a target object which is monitored by the monitoring device;
at least one ultrasonic device, wherein each ultrasonic device is configured to perform an ultrasonic examination on the target object and generate ultrasonic information of the target object; and
the medical information display system mentioned above according to the first aspect.

Specifically, a fourth aspect of this disclosure provides a medical system, which includes:
the medical information display system mentioned above according to the second aspect;
at least two of the monitoring device, the examination device, the treatment device, and the camera device, wherein the monitoring device is configured to acquire the vital sign information of the target object which is monitored by the monitoring device, the examination device is configured to exam the target object and generate the examination information of the target object, and the treatment device is configured to treat the target object and output the treatment information, the camera device is configured to acquire the imaging information of the target object.

The medical information display system according to the first aspect of this disclosure, can simultaneously present the vital sign information and ultrasonic information of the interested target object to the user, and the ultrasonic information is displayed with a timestamp or the vital sign information and ultrasonic information are correlatively displayed based on the timestamp, thereby making it more convenient for the user to view and analyze the two types of data, improving the viewing and analysis efficiency.

The medical information display system according to the second aspect of this disclosure, can store the medical information of the target object with its storage device, wherein the medical information includes at least two types of vital sign information, examination information, treatment information, and imaging information; and can also display detailed information of the medical information of the selected target object on the display device, thereby making it more convenient for the user to view and analyze at least two types of medical information of the selected target object, improving viewing and analysis efficiency.

### BRIEF DESCRIPTION OF THE DRAWINGS

In order to provide a clearer explanation of the technical solution in the embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.
FIG. 1 shows a schematic block diagram of a medical information display system in one embodiment of this disclosure.
FIG. 2 shows a schematic diagram of a display observation interface provided by a display device of a medical information display system in one embodiment of this disclosure.
FIG. 3 shows a schematic diagram of a medical information display system in one embodiment of this disclosure, simultaneously displaying ultrasonic information and vital sign information.
FIG. 4 shows a schematic diagram of a medical information display system in another embodiment of this disclosure, displaying abbreviated information of alarm event and abbreviated information of ultrasonic information.
FIG. 5 shows a schematic diagram of a medical system in one embodiment of this disclosure.
FIG. 6 shows a schematic diagram of a medical system in another embodiment of this disclosure.
FIG. 7 shows a schematic structure diagram of the structure of a medical information display system in another embodiment of this disclosure.
FIG. 8 shows a schematic diagram of a medical information display system in one embodiment of this disclosure, simultaneously displaying video data and monitoring data.
FIG. 9 shows a schematic diagram of a medical information display system in one embodiment of this disclosure when reviewing historical data.
FIG. 10 shows a schematic diagram of a medical information display system in one embodiment of this disclosure when simultaneously displaying video data and ultrasonic image.

### DESCRIPTION OF THE PREFERRED EMBODIMENTS

In order to make the purpose, technical solution, and advantages of this disclosure more apparent, the following refers to the attached drawings to describe in detail the exemplary embodiments according to this disclosure. Obviously, the described embodiments are only some of the embodiments of this disclosure, not all of them. It should be understood that this disclosure is not limited by the exemplary embodiments described here. Based on the embodiments described in this disclosure, all other embodiments obtained by those skilled in the art without creative labor should fall within the protection scope of this disclosure.

In the following description, a large number of specific details are provided to provide a more thorough understanding of this disclosure. However, it is evident to those skilled in the art that this disclosure can be implemented without one or more of these details. In other examples, in order to avoid confusion with this disclosure, some well-known technical features in the art have not been described.

It should be understood that this disclosure can be implemented in different forms and should not be limited to the embodiments proposed here. On the contrary, providing these embodiments makes the disclosure thorough and complete, and fully conveys the scope of this disclosure to those skilled in the art.

The purpose of the technical term used here is only to describe specific embodiments and is not a limitation of this disclosure. When used here, the singular forms of "a", "an", and "said/the" are also intended to include the plural form, unless the context clearly indicates another way. It should also be understood that the terms "comprise" and/or "include", when used in this disclosure, determine the presence of the features, integers, steps, operations, elements, and/or components, but do not exclude the presence or addition of one or more other features, integers, steps, operations, elements, and/or components. When used here, the term "and/or" includes any and all combinations of related listed items.

In order to fully understand this disclosure, a detailed structure is proposed in the following description to elucidate the technical solution proposed by this disclosure. The optional embodiments of this disclosure are described in detail below, however, in addition to these detailed descriptions, this disclosure may also have other embodiments.

Specifically, the following provides a detailed explanation of a medical information display system of this application in conjunction with the accompanying drawings. Without conflict, the features in the following embodiments and implementations can be combined with each other.

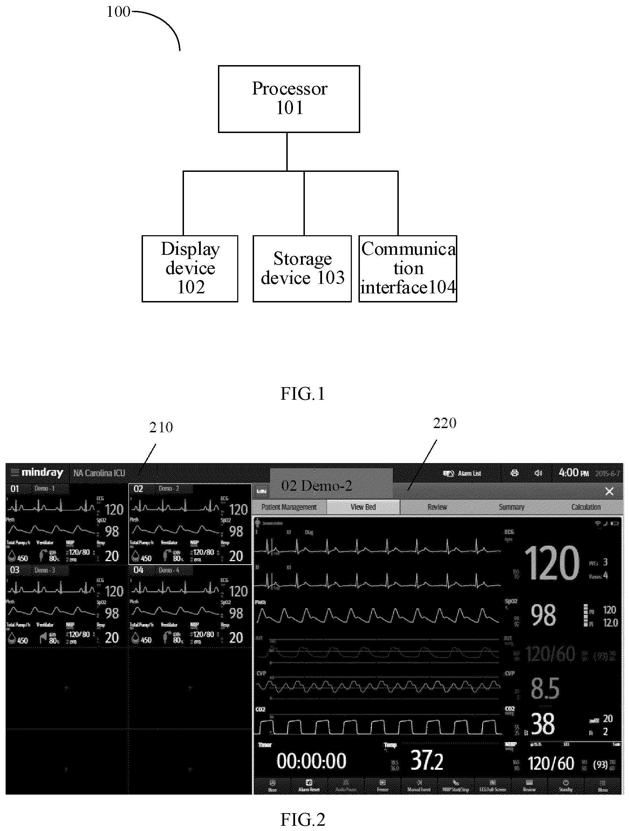

Below, a medical information display system in one embodiment of this disclosure is described with reference to FIGS. 1 to 4.

Medical information display system 100 in the embodiment of this disclosure includes but is not limited to a monitoring device, a central station, any suitable PC, and the likes. The following embodiments mainly use the central station as an example to explain.

As an example, as shown in FIG. 1, the medical information display system 100 includes one or more processors 101, a display device 102, a storage device 103, and a communication interface 104. These components are interconnected through a bus system and/or other forms of connection mechanisms (not shown). It should be noted that the components and structure of the medical information display system 100 shown in FIG. 1 are only exemplary and not restrictive. When necessary, the medical information display system 100 may also have other components and structures.

The storage device 103 is configured to store various data and executable program instructions, such as system program instructions of the medical information display system, various application program instructions, or algorithms for implementing various specific functions. The storage device 103 is configured to store vital sign information which is acquired by the monitoring device and ultrasonic information which is generated by the ultrasonic device.

The storage device 103 may include one or more computer program products, which may further include various forms of computer-readable storage media, such as volatile memory and/or non-volatile memory. The volatile memory may include, for example, random access memory (RAM) and/or cache memory. The non-volatile memory may include, for example, read-only memory (ROM), hard disk, flash memory, etc. When the medical information display system obtains data information sent by various devices such as monitors, ventilators, ultrasonic devices, camera devices, etc., any data that needs to be stored locally can be stored in the storage device 103.

The processor 101 can be a central processing unit (CPU), image processing unit (GPU), specialized integrated circuit (ASIC), field programmable gate array (FPGA), or other forms of processing unit with data processing and/or instruction execution capabilities, and can also control other components in the medical information display system to perform the desired functions. For example, the processor can include one or more embedded processors, processor cores, microprocessors, logic circuits, hardware finite state machines (FSM), digital signal processors (DSP), image processing units (GPU), or a combination of them.

In one example, the medical information display system 100 also includes a communication interface 104 for communication between various components in the medical information display system, as well as between various components of the medical information display system and other devices outside the system (such as monitoring devices, ultrasonic devices, etc.), for example, at least for communication between the medical information display system and a monitoring device, for communication between the medical information display system and the ultrasonic device, to facilitate information exchange between the medical information display system, the monitoring device, and the ultrasonic device.

The communication interface 104 can be any known communication protocol interface, such as a wired interface or a wireless interface, wherein the communication interface 104 can include one or more of serial ports, USB interfaces, Ethernet ports, Wi-Fi, wired networks, DVI interfaces, device integrated interconnection modules, or other suitable various ports, interfaces, or connections. The medical information display system 100 can also be connected to wireless networks based on communication standards, such as Wi-Fi, 2G, 3G, 4G, 5G, or a combination of them. In an exemplary embodiment, the communication interface 104 receives broadcast signals or broadcast related information from an external broadcast management system through a broadcast channel. In an exemplary embodiment, the communication interface 104 further includes a Near Field Communication (NFC) module to facilitate short range communication. For example, the NFC module can be implemented based on radio frequency identification (RFID) technology, infrared data association (IrDA) technology, ultra-wideband (UWB) technology, Bluetooth (BT) technology, and other technologies.

In one example, when a plurality of monitoring devices exists, the communication interface 104 communicatively connects with the plurality of monitoring devices, the medical information display system 100 can obtain vital sign information of target objects which are connected with each of the plurality of monitoring devices through the communication interface 104. When a plurality of ultrasonic devices exists, the communication interface 104 communicatively connects with the plurality of ultrasonic devices, and the medical information display system 100 can obtain ultrasonic information from the plurality of ultrasonic devices through the communication interface 104.

In another example, the communication interface 104 communicates with the monitoring device, which is configured to receive ultrasonic information from the ultrasonic device which is in communication with the monitoring device, as well as to receive vital sign information which is outputted from the monitoring device.

In another example, when the communication interface 104 is in communication with the ultrasonic device, the communication interface 104 is configured to receive ultrasonic information which is stored locally by the ultrasonic device. The ultrasonic information stored locally can be real-time acquired ultrasonic information, or the ultrasonic information stored locally can be historical data of ultrasonic information, etc.

In one example, the communication interface 104 is also configured to transmit the vital sign information and/or ultrasonic information stored in the storage device 103 to the monitoring device, so that the monitoring device displays the vital sign information and/or ultrasonic information, and the user can view the corresponding review information of the target object on the monitoring device, such as a bedside monitor. The review information is stored in the storage device 103 of the medical information display system 100, which can reduce the storage space occupied by directly storing this information in the monitoring device.

In one example, the medical information display system 100 also includes an input device (not shown), which may be a device used by a user to input instructions, and may include one or more of a keyboard, a trackball, a mouse, a microphone, a touch screen, and the likes, or may be an input device composed of other control buttons. For example, the user can input instructions to view various data information through the input device.

The medical information display system 100 of the embodiment of this disclosure also includes an output device that can output various information (such as images or sounds) to an external device (such as a user), and can include one or more of the display devices 102, a speaker, etc.

In one example, the medical information display system 100 also includes one or more display devices 102, which can be a touch screen, liquid crystal display screen, etc., built-in to the medical information display system 100, as well as can be an independent display device, such as a liquid crystal display device, a television, etc., independent of the medical information display system 100. Optionally, the one or more display devices 102 can also be a display screen on an electronic device, such as a mobile phone, tablet, etc.

The display device 102 is at least configured to display the vital sign information and the ultrasonic information of one or more target objects. The display device 102 can display the vital sign information and ultrasonic information obtained by the processor 101. The medical information display system 100 also includes a user interface, through which the user of the medical information display system can control the operation of the medical information display system. The user interface may be provided by the display device 102, which may include a touch screen that allows the user to input operation instructions to the medical information display system from the display device 102, and/or include one or more control panels, etc., through which the user can control the operation of the medical information display system.

In one example, the processor 101 is configured to execute the program instructions stored in the storage device 103, enabling the processor 101 to perform following actions: obtaining the vital sign information acquired by the monitoring device and ultrasonic information generated by the ultrasonic device through the communication interface 104, and controlling the storage device 103 to store the vital sign information and the ultrasonic information according to a timestamp; controlling the display device 102 to provide a display observation interface, which includes a plurality of display areas, each display area correspondingly displays at least part of the vital sign information of one target object. For example, the display observation interface 210 shown in FIG. 2 provides four display areas, which simultaneously display at least part of the vital sign information of four target objects.

The processor 101 controls the storage device 103 to store the vital sign information and ultrasonic information according to the timestamp. For example, the processor 101 controls the storage device 103 to store the vital sign information and the ultrasonic information to a database according to the timestamp and identification information of the target object.

Optionally, the ultrasonic information includes identification information of the target object and time information of the ultrasonic examination, and the ultrasonic information also includes at least one of an ultrasonic image, an ultrasonic examination report, and a measurement result based on ultrasonic image(s). Optionally, the ultrasonic image can be a single image or an ultrasonic video of a plurality of consecutive frames of ultrasonic images, etc., as well as can be a real-time ultrasonic image when the ultrasonic device is examining the target object, or a historical ultrasonic image.

The vital sign information includes identification information of the target object, as well as at least one of: vital sign parameter information of the target object, and alarm event information of the target object. Optionally, the vital sign parameter information includes but is not limited to monitoring information of one or more parameters, such as EEG, electrocardiogram, heart rate, blood pressure, central venous pressure (CVP), blood oxygen, body temperature, and respiratory rate. Optionally, the vital sign parameter information includes waveform and/or numerical values, trend plots, etc., of vital sign parameters.

Optionally, the identification information of the target object includes, but is not limited to, one or more of: such as patient support number of target object, name of target object, identification code of target object (such as ID number or medical insurance card number, etc.). The identification information of the target object can be configured to associate the ultrasonic information and the vital sign information of the same target object, so as to avoid the occurrence of inconsistencies that may lead to misjudgment of doctor on patient condition.

It is worth to mention that, storing according to a timestamp, means that storing various information according to generation time of the various information, so that the user can retrieve the information they want within a preset time period when reviewing.

Furthermore, the user can operate one display area of the plurality of display areas, such as opening corresponding detail interface of the interested target object through a mouse or touch screen. The detail interface can be configured to display real-time information and review information of the interested target object. Specifically, the processor 101 is also configured to control the display device 102 to display a detail interface of the interested target object corresponding to the selected display area when a selection instruction for one of the plurality of display areas is obtained (such as based on clicking the corresponding display area through a mouse or touch screen). The detail interface is set with a review hotkey, such as "Review" hotkey on the detail interface 220 shown in FIG. 2. Optionally, the detail interface 220 is also set with a real-time information viewing hotkey, such as "View Bed" hotkey. Optionally, the detail interface 220 can be located on one side of the display observation interface 210 and does not cover the plurality of display areas displayed on the display observation interface 210, so that while viewing the interested target object, some vital sign information of other target objects can also be observed.

In addition to displaying the real-time information viewing hotkey and the review hotkey, the detail interface can also have corresponding hotkeys for patient management, allowing for switching between the real-time viewing, review, and patient management interfaces. It should be noted that real-time and review are two relative concepts. For example, in the example shown in FIG. 2, "View Bed" hotkey is configured to provide a real-time interface, presenting real-time vital sign information of the target object; while "Review" and "Summary" hotkeys are both used as review hotkeys to provide review information from different review perspectives of the target object. For example, the detail interface can have a plurality of review hotkeys, each is configured to present review information from different review perspectives.

In one example, the processor 101 is also configured to control the display device 102 to display a real-time interface of the interested target object on the detail interface 210 when a viewing instruction for the real-time information viewing hotkey is obtained. The real-time interface is configured to display real-time information of the interested target object, such as real-time vital sign parameter information, as shown in FIG. 2.

Furthermore, the processor 101 is further configured to control the display device 102 to display a review interface of the interested target object on the detail interface 220 when a review instruction for the review hotkey is obtained. The review interface is configured to display review information of the interested target object, including the vital sign information and the ultrasonic information. Among them, the ultrasonic information is displayed with the timestamp, or the vital sign information and ultrasonic information are correlatively displayed based on the timestamp, making it more convenient for the user to view and analyze the two types of data, improving viewing and analysis efficiency, and helping the doctor to diagnose the current state of illness for the patient according to the trend of review information and the current condition of the patient, so as to develop more reasonable treatment strategies.

Optionally, the review information includes but is not limited to at least one type of: a table of vital sign parameter information in the vital sign information, ultrasonic image review of ultrasonic information, list and detail review of alarm event, and parameter waveform review of vital sign parameters.

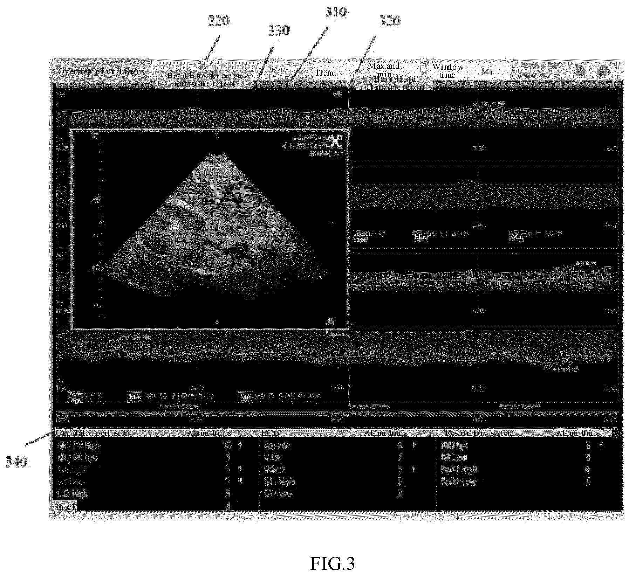

In one example, the processor 101 is also configured to control the display device 102 to display the corresponding the review information in a chronological order within a preset time period on the review interface when a review instruction for the review information within the preset time period is obtained. Optionally, the preset time period can be any suitable time, and a control element for setting the preset time period is set on the review interface (such as the window time in FIG. 3). The processor 101 can be configured to, when obtaining an operation instruction for the control element, control to set the time period corresponding to the operation instruction as the preset time period. The range of this preset time period can be reasonably set according to requirements of the user, and no specific limitation is made herein.

The review interface displays a time marker representing the chronological order (such as timelines 310 and 340 shown in FIG. 3), and ultrasonic examination indications 320, such as ultrasonic examination indications for heart/lung/abdomen ultrasonic report and for heart/cranial ultrasonic report as shown in FIG. 3, are provided at time points of the time marker, wherein which time points correspond to the examination time of each ultrasonic information. The ultrasonic examination indication can be characterized by graphics and/or text. Optionally, each ultrasonic examination indication can also indicate a type of examined portion of the patient in the corresponding ultrasonic report, such as heart/lung/abdomen, heart/skull, etc., making it easy for the user to quickly select the ultrasonic report wanted to view based on this indication.

In one example, the review information also includes the alarm event of the target object, and the processor 101 is configured to provide an alarm event indication at the time point corresponding to the alarm event of the target object on the time marker, with the ultrasonic examination indication. The ultrasonic examination indication and the alarm event indication are displayed differently to facilitate the user to distinguish the events identified by each marker, and the alarm event indication and ultrasonic examination indication are correlatively displayed. This allows the user to easily obtain ultrasonic information when investigating the causes of alarm events, so as to analyze the patient condition based on the two types of data.

For example, as shown in FIG. 3, the time marker can include two timelines, the ultrasonic detection indication and alarm event indication can be marked on different timelines. For example, the ultrasonic examination indication can include a graph marker and a text marker. When receiving the ultrasonic examination indication from the user, the processor can control to provide a linear marker on the timeline. Through this linear marker, the user can intuitively obtain the time indicated by the ultrasonic examination indication. For example, the ultrasonic detection indication and the alarm event indication can also be marked on the same timeline and displayed differently in different colors and/or shapes, text, etc.

In one example, the processor 101 is also configured to, when obtaining a selection instruction for one ultrasonic examination indication, control the display device 102 to display detailed information of the ultrasonic information corresponding to the selected ultrasonic examination indication. For example, the selection instruction can be generated by placing a cursor at the ultrasonic examination indication, or clicking the ultrasonic examination indication by a mouse. The display device 102 can display detailed information of the ultrasonic information, ultrasonic images, etc., corresponding to the selected ultrasonic examination indication in an ultrasonic window. Optionally, the ultrasonic window can be located at the corresponding ultrasonic examination indication. Optionally, as shown in FIG. 3, the ultrasonic examination indication can include a graph marker and a text marker. When the linear marker indicating the time appears, the ultrasonic window 330 can be located on one side of the linear marker. The ultrasonic window 330 for displaying ultrasonic information is set with a close hotkey, and the processor 101 is also configured to control the ultrasonic window 330 to be closed, in response to an operation instruction for the close hotkey.

For example, as shown in FIG. 3, the review interface is also set with control elements for selecting a display style of review information, such as the control element corresponding to "Vital sign Overview" shown in FIG. 3. The control element is set with a plurality of options. The processor 101 is configured to, when obtaining a selection instruction for one target option in the control element, control the review information to be displayed in a preset style corresponding to the target option on the review interface. For example, when the "Vital sign Overview" option is selected, the review interface displays vital sign overview information, ultrasonic examination indication, and alarm event indication, etc., within the preset time period.

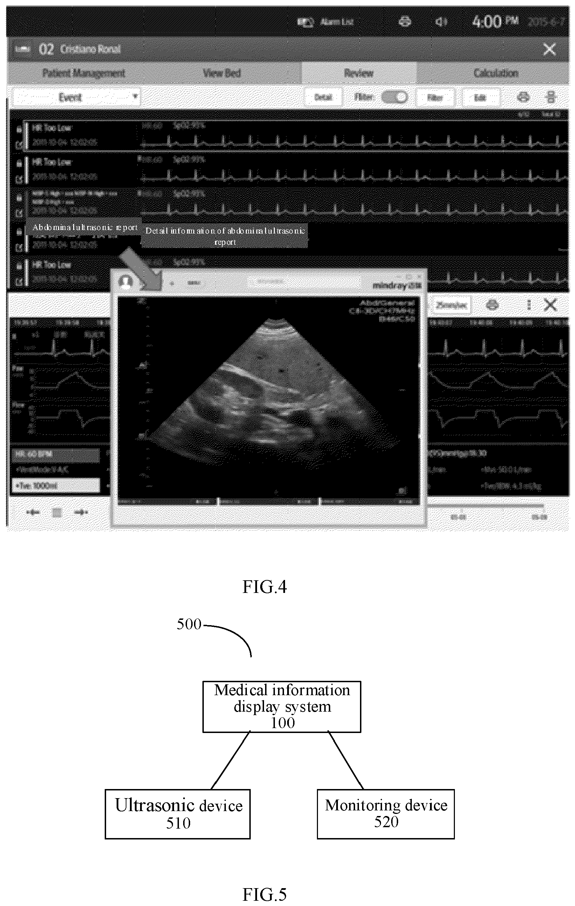

In one example, as shown in FIG. 4, the processor 101 is also configured to, when obtaining a review instruction for the review information within a preset time period, control the display device 102 to display abbreviated information of the ultrasonic information within the preset time period in a list format on the review interface. The abbreviated information of the ultrasonic information includes ultrasonic examination indication and time information of the ultrasonic examination. Among them, each ultrasonic examination indication can only indicate a presence of one piece of ultrasonic examination information, and can also indicate a type of examined portion of the patient in the corresponding ultrasonic report, such as heart/lung/abdomen, heart/skull, etc., so that the user can quickly select the ultrasonic report they want to view based on this indication. By displaying the abbreviated information of ultrasonic information, it is convenient for the user to browse the abbreviated information of a plurality of ultrasonic reports at the same time, thus facilitating to determine detailed information of which ultrasonic report is wanted based on the abbreviated information.

The processor 101 is also configured to, when obtaining an ultrasonic detail display instruction for one piece of abbreviated information of the ultrasonic information which is displayed on the review interface (such as the ultrasonic detail display instruction which is generated by clicking one piece of abbreviated information of the ultrasonic information based on a mouse or touch screen), control the display device 102 to display the detailed information of the selected ultrasonic information on the review interface, or control the display device 102 to display the ultrasonic window in the form of a floating window on the current interface of the detail interface. The ultrasonic window displays the detailed information of the ultrasonic information, which at least includes the ultrasonic image, such as the ultrasonic image shown in FIG. 4. Optionally, the detailed information of the ultrasonic information can also include an ultrasonic examination report, or a measurement result based on ultrasonic image(s), etc. In other examples, the ultrasonic window can also be embedded into the detail interface, and the layout of other elements in the interface can be adjusted accordingly to achieve simultaneous display.

The medical information display system, such as a central station, combines the vital sign information and the ultrasonic information of the same patient and provides a review function in a chronological order. For example, the processor 101 is also configured to control the display device 102 to display the abbreviated information of the alarm event within the preset time period in a list format on the review interface, wherein the abbreviated information of the alarm event and the abbreviated information of the ultrasonic information are arranged in a chronological order. For example, the review interface is also set with control elements for selecting the display style of review information, such as the control element corresponding to "Event" shown in FIG. 4. The control element is set with a plurality of options, and the processor 101 is configured to, when obtaining a selection instruction for one of the plurality of options in the control element, control to display the review information in the preset style corresponding to the target option on the review interface. For example, as shown in FIG. 4, when the "Event" option is selected, the display device 102 is controlled to display the abbreviated information of ultrasonic information and the abbreviated information of the alarm event within the preset time period in a list format on the review interface. By displaying this information in this way, the efficiency for the user to view and analysis is improved.

In one example, the processor 101 is also configured to control the display device 102 to provide ultrasonic graph on the detail interface of the interested target object. Optionally, the ultrasonic graph can be any graph, symbol, or text that can represent ultrasound, such as a circular shape. The processor 101 is also configured to, when obtaining an input instruction for the ultrasonic graph, control the display device 102 to display the ultrasonic window in the form of a floating window on the current interface of the detail interface, and the ultrasonic information is displayed in the ultrasonic window. The ultrasonic information at least includes an ultrasonic image, or can also include an ultrasonic examination report, a measurement result based on ultrasonic image(s), etc.

It is worth mentioning that the interested target object may have one or more ultrasonic examinations within a preset time period, and the processor 101 can also be configured to control the display device 102 to display one or more pieces of ultrasonic information of the same interested target object on the review interface or in the ultrasonic window.

Furthermore, there is one or more camera devices installed inside a ward where each target object locates, wherein the camera device(s) is/ are configured to capture imaging information of each target object, such as video information, and/or image information, etc. In one example, the detail interface of the interested target object is set with a video access hotkey. When the review interface of the interested target object is displayed on the detail interface, the processor 101 is also configured to, when obtaining a video viewing instruction for the video access hotkey, obtain historical video information of the interested target object within a preset time period, and control the display device 102 to display the historical video information of the interested target object. The preset time period can be reasonably set according to requirement of the user, and it can also be the same or different from the aforementioned preset time period of the review information. Among them, the historical video information can be displayed in a video display window provided by the display device 102, and the video display window can be displayed in the form of a floating window on the current interface of the detail interface, or can also be displayed on the review interface of the interested target object. Optionally, the display device 102 can also provide a control element for controlling a playback speed of historical video information, as well as a control element for dragging and dropping the historical video information.

Optionally, the historical video information and the ultrasonic information are correlatively displayed based on the timestamp. The historical video information is configured to provide examination operation information for the interested target object while acquiring the ultrasonic information, so as to facilitate the user to determine whether a scanning acquisition process of the ultrasonic information is reasonable based on the examination operation information, and so as to provide guidance for subsequent ultrasonic examination of the interested target object for the user through this examination operation information.

In one example, the detail interface of the interested target object is set with a video access hotkey. When the real-time interface of the interested target object is displayed on the detail interface, the processor 101 is also configured to, when obtaining a video viewing instruction for the video access hotkey, obtain current real-time video information of the interested target object, and control the display device 102 to display the current real-time video information of the interested target object. Wherein the real-time video information can be displayed in a real-time video display window provided by the display device 102. The real-time video display window can be displayed in the form of a floating window on the current interface of the detail interface, or can also be displayed in the real-time interface of the interested target object.

In one example, each display area is set with a video access hotkey, and the processor 101 is further configured to, when obtaining a video viewing instruction for the video access hotkey, obtain current real-time video information of the target object corresponding to the display area where the video access hotkey is located, and control the display device 102 to display the real-time video information. Alternatively, the display observation interface is set with a video access hotkey, and the processor 101 is also configured to, when obtaining a video viewing instruction for the video access hotkey, obtain current real-time video information of one or more target objects, and control the display device 102 to simultaneously display the real-time video information of the one or more target objects.

In one example, when the ultrasonic device exams the interested target object, the real-time video information is configured to display the examination process of the interested target object. The processor 101 is also configured to obtain examination operation guidance information required for the ultrasonic examination of the ultrasonic device, and transmit the examination operation guidance information to the ultrasonic device through the communication interface 104, so as to enable the operator of the ultrasonic device to conduct the ultrasonic examination on the patient based on the examination operation guidance information, for obtaining necessary ultrasonic information. For patients in isolation wards, doctor outside the ward can use this method to guide the operator in isolation wards to perform ultrasonic examinations on patients and improve examination efficiency. Optionally, the examination operation guidance information can be provided through real-time voice and/or video examination operation guidance provided by a medical information display system, for example.

In one example, the processor 101 is also configured to control to display at least one of followings in the form of a bullet-screen on the video information: an alarm event, ultrasonic information, and at least some vital sign information. When the displayed video information is a real-time video, the real-time vital sign information and/or ultrasonic information can be displayed in the form of a bullet-screen on the video information. When the displayed video information is a historical video, the historical vital sign information and/or historical ultrasonic information corresponding to the specific time point can be displayed on the historical video information based on the selection of the specific time point on the timeline on the review interface.

At this point, the description of the components of the medical information display system of this disclosure is completed. However, it can be imagined that in addition to the components described in the embodiments of this disclosure, the medical information display system of this application can also include more or fewer components.

In summary, according to the embodiment of this disclosure, the medical information display system can simultaneously present the vital sign information and ultrasonic information of the interested target object to the user, and the ultrasonic information is displayed with the timestamp or the vital sign information and ultrasonic information are correlatively displayed based on the timestamp, thereby making it more convenient for the user to view and analyze the two types of data, improving the viewing and analysis efficiency.

Referring to FIG. 5, this disclosure also provides a medical system 500, which includes at least one monitoring device 520, wherein each monitoring device is configured to acquire vital sign information of the target object which is monitored by the monitoring device; at least one ultrasonic device 510, wherein each ultrasonic device is configured to perform an ultrasonic examination on the target object and generate ultrasonic information of said target object; and the medical information display system 100 of the aforementioned embodiments.

Specifically, some detailed descriptions of the medical information display system 100 can refer to the aforementioned embodiments and are repeated here.

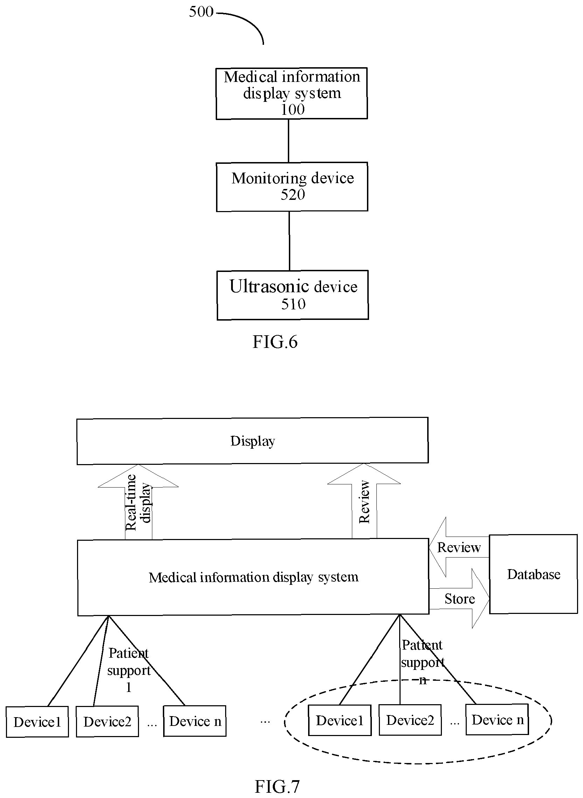

The medical information display system 100, such as a central station, can connect a plurality of target objects, that is, can connect a plurality of monitoring devices and ultrasonic devices simultaneously. There are various ways to connect devices. In one way, as shown in FIG. 5, two devices are independently connected with the medical information display system 100, such as a central station. The ultrasonic device transmits real-time ultrasonic information to the central station. In another way, as shown in FIG. 6, the ultrasonic device is connected with the central station through a monitoring device, and the ultrasonic information is transferred and transmitted to the central station through the monitoring device. In a further way, any one or more ultrasonic devices among a plurality of ultrasonic devices, stores/store ultrasonic information locally. When the ultrasonic device is connected to the network, the locally stored ultrasonic information is transmitted to the central station through the network.

In one example, as shown in FIG. 5, the medical information display system 100, such as a central station, can communicatively connect with one or more monitoring devices and one or more ultrasonic devices. Each monitoring device is configured to acquire vital sign information of the target object which is monitored by the monitoring device and transmit the vital sign information to the medical information display system 100. Each ultrasonic device is configured to perform an ultrasonic examination on the target object and generate ultrasonic information of the target object, and transmit the ultrasonic information to the medical information display system 100.

The medical information display system 100, such as the central station, is configured to store the vital sign information which is transmitted by the monitoring device and the ultrasonic information which is transmitted by the ultrasonic device, according to the timestamp. For example, the medical information display system 100 is further configured to store the ultrasonic information and vital sign information in the database, and retrieve them from the database according to instructions when necessary, so as to facilitate the user to review the ultrasonic information and vital sign information.

Due to the fact that the medical system 500 of this embodiment has aforementioned medical information display system 100, it has the same advantages.

The medical information display system in another embodiment of this disclosure is described below with reference to FIGS 1, 7, and 10.

Firstly, as shown in FIG. 1, the medical information display system 100 of this disclosure includes one or more processors 101, a display device 102, a storage device 103, and a communication interfaces 104. These components are interconnected through a bus system and/or other forms of connection mechanism (not shown). It should be noted that the components and structure of the medical information display system 100 shown in FIG. 1 are only exemplary and not restrictive. As needed, the medical information display system 100 may also have other components and structures. The detailed description of the above components of the medical information display system 100 in this embodiment can refer to the description of the previous embodiment.

In one example, the communication interface 104 of the medical information display system 100 is configured to communicatively connect at least one of the following devices: a monitoring device, an examination device, a treatment device, and a camera device. Among them, the communication interface 104 can also communicatively connect with devices which are associated with a plurality of target objects, for example, each target object can be associated with at least two devices such as the monitoring device, the examination device, the treatment device, and the camera device. For example, as shown in FIG. 7, the medical information display system, such as a central station, can communicatively connect with a plurality of target objects (such as patient support 1... patient support n), and each target object is associated with a plurality of devices such as device 1, device 2, device n, etc. The medical information display system can obtain medical information transmitted by these devices and store the medical information in a database according to a timestamp. When necessary, the processor 101 can be configured to read corresponding review information in the database in response to a review instruction. The display device 102 of the medical information display system, such as a display screen, can also display the obtained medical information or review information in real-time.

The monitoring device is configured to acquire vital sign information of the target object which is monitored by the monitoring device, and the monitoring device includes but is not limited to a bedside monitor or a wearable monitor.

The examination device is configured to examine the target object and generate examination information of the target object. The examination device can be an imaging examination device, for example, the examination device includes at least one type of ultrasonic device and X-ray imaging device, or any other suitable examination device.

The treatment device is configured to treat the target object and output treatment information. Optionally, the treatment device includes at least one type of a ventilator, defibrillator, infusion pump, oxygen therapy instrument, anesthesia machine and extracorporeal membrane oxygenation (ECMO), or other device that can treat the patient.

The camera device is configured to acquire imaging information of the target object, such as video information and/or image information , as well as audio information. The camera device, such as a camera head, can be any device with image acquisition functions, and each target object can correspond to one or more camera devices.

In one example, the processor 101 is also configured to control the storage device 103 to store the vital sign information, examination information, treatment information, and imaging information according to a timestamp (such as indexed by time) after obtaining the medical information through the communication interface 104, so that when the subsequent user review the medical information, the user can retrieve information for the desired time period.

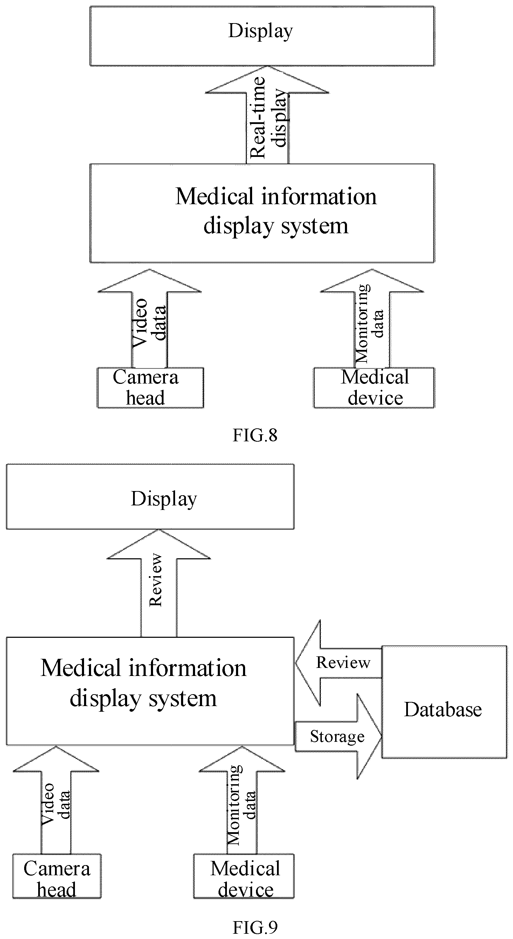

For example, as shown in FIG. 9, a medical information display system (such as a processor 101 of the medical information display system) is also configured to store medical information in a database according to a timestamp (such as indexed by time) after obtaining the medical information through communication interface 104. When needing to review historical data, the medical information display system provides a video access, and the processor 101 can also be configured to read the medical information stored in the database for a preset time period, and control the display device 102 to display the medical information, such as to display the medical information concentratedly in the display interface of the display device 102 through text, charts, etc. Medical information includes but is not limited to video information, vital sign information, medical imaging information , etc., within the preset time period.

Furthermore, the storage device 103 of the medical information display system 100 is configured to store executable program instructions and medical information of a target object, wherein the medical information includes at least two types of vital sign information, examination information, treatment information, and imaging information. The number of target objects can be one or more. By storing this information through the storage device 103, the user can easily access and view it in the future, when necessary, so as to analyze the patient condition.

Furthermore, one or more processors 101 are configured to execute the program instruction stored in storage device 103, to enable the processor 101 to perform the following steps: obtaining the medical information of a plurality of target objects through the communication interface 104, wherein the medical information of each target object includes at least two types of vital sign information, examination information, treatment information, and imaging information; controlling the storage device 103 to store the obtained medical information; and controlling the display device 102 to display the medical information of the plurality of target objects, and when receiving a selection instruction for one target object, controlling the display device 102 to display detailed information of the medical information of a selected target object; and displaying the medical information of the selected target object on the display device 102. For example, the display device 102 can display at least two types of medical information of the interested target object in text, charts, and other ways. Thus, the user can more conveniently view and analyze at least two types of medical information of the selected target object, improving the efficiency of viewing and analysis, and helping the doctor to diagnose the current state of illness for the patient according to the trend of review information and the current condition of the patient, so as to develop more reasonable treatment strategies.

For example, as shown in FIG. 8, the processor 101 of the medical information display system can be configured to control the display device 102 to display video information of the selected target object and monitoring information of other devices. The user can determine the patient state through the video, combined with real-time parameters, waveforms, alarms, medical images, etc. measured by other medical devices.

Optionally, the vital sign information includes at least one type of the following information: vital sign parameter information of the target object, and alarm event information.

Optionally, the examination information includes at least one type of the following information: medical imaging information, medical examination report, medical diagnostic result, etc., or may also include other information related to the examination device. For example, when the examination device is an ultrasonic device, the medical imaging information can be an ultrasonic image, and the medical examination report includes an ultrasonic examination report, and the medical diagnostic result includes a diagnostic result made by a doctor based on an ultrasonic image.

In one example, the treatment information includes at least one type of the following information: administration information, ventilation parameter information, defibrillation treatment parameter information. The administration information can be administration information which is obtained from an infusion pump, such as administration dose, infusion flow rate, etc. The ventilation parameter information can be respiratory related parameter information which is obtained from a ventilator, such as pressure waveform, volume waveform or flow velocity waveform, tidal volume, PEEP, etc. Defibrillation treatment parameter information can be parameter information which is obtained from a defibrillator, such as defibrillation time, electrocardiogram parameters, etc.

In one example, the imaging information acquired by the camera device includes at least one type of the following information: video information of the target object and image information of the target object. The video information of the target object and/or the image information of the target object include/includes at least one type of: body surface information of the target object, dynamic information of the target object, examination operation information or treatment operation information which is performed on the target object. Through the imaging information acquired by the camera device, the user can observe the patient state during a past time period, which helps the doctor to diagnose the current state of illness for the patient according to the trend of review information and the current condition of the patient, so as to develop more reasonable treatment strategies.

The medical information display system of this embodiment can combine and centrally display medical data (such as ultrasonic information, vital sign information, and treatment parameter) with video and/or image data (such as video data), such as combining and centrally displaying the ultrasonic data, vital sign information, treatment parameter and patient state information (such as complexion, facial expression and body state of patient,) at the same moment, so as to enable the doctor to easily obtain information from a plurality of dimensions of patient and make timely determination based on this information.

In one example, the processor 101 is also configured to control the display device 102 to provide a display observation interface, which includes a plurality of display areas, each display area correspondingly displays at least some medical information of a target object. For example, when connecting with a monitoring device, each display area correspondingly displays at least some vital sign information acquired by the monitoring device, such as at least some real-time vital sign parameter information of the current target object, such as waveform and/or numerical value. Alternatively, when connecting with a ventilator or anesthesia machine, each display area correspondingly displays respiratory related ventilation parameter information obtained from the ventilator, such as pressure waveform, volume waveform or flow velocity waveform, tidal volume, PEEP, etc.

In one example, the processor 101 is also configured to, when receiving a selection instruction for one of the plurality of display areas, control the display device 102 to display a detail interface of the interested target object corresponding to the selected display area. The detail interface includes a plurality of display interfaces, each of which is configured to display one type of medical information. Optionally, the plurality of display interfaces can be a plurality of display areas located on the same display interface, or the plurality of display interfaces can each correspond to a single display window, which can float on the current interface of the display observation interface.

In one example, the display device 102 provides a display observation interface, which includes a plurality of display areas. Each display area correspondingly displays at least some medical information of one target object, and each display area also correspondingly displays an icon of a medical device associated with one target object. When the processor 101 obtains an operation instruction for the icon of one or more medical devices in the display area of the interested target object, the processor 101 controls the display device 102 to display medical information of one or more corresponding medical devices. Among them, the icons of different medical devices can have different patterns, or the icons can also include patterns and text configured to describe the type of medical device.

In one example, the processor 101 is also configured to control the display device 102 to provide a display observation interface, which includes a plurality of display areas, each display area correspondingly displays at least some medical information of one target object. When a selection instruction for one of the plurality of display areas is obtained to select one target object, the processor 101 is also configured to control the display device 102 to display a detail interface of the interested target object corresponding to the selected display area. The detail interface includes a plurality of display interfaces, and one display interface is configured to display at least two types of medical information.

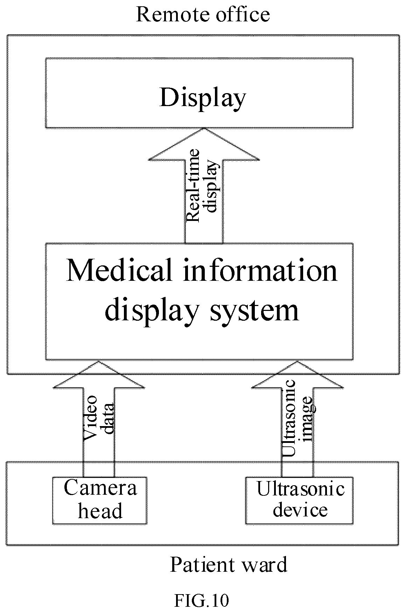

As shown in FIG. 10, the medical information display system can remotely communicatively connect with a camera device, an ultrasonic device, and a database. In this scenario, the remote office can directly view the state of patients in the ward or operating room, such as body, complexion, facial expression, etc., through the video information (also known as video data in this disclosure) captured and transmitted by the camera device. At the same time, the ultrasonic information, such as ultrasonic imaging information (including but not limited to ultrasonic images), ultrasonic examination report, vital sign information, and other data, can be viewed at the same time. For expert doctors in remote offices (such as different hospitals, cities, etc.), this remote method can guide doctors in wards or operating rooms.

As shown in FIG. 10, the medical information display system of this disclosure embodiment can also display imaging information (such as video information) and ultrasonic information, on the same screen. For example, when the examination device includes an ultrasonic device, the processor 101 is also configured to obtain real-time imaging information of the target object acquired by the camera device and real-time ultrasonic imaging information of the target object measured by the ultrasonic device through the communication interface 104, and control the display device 102 to display real-time imaging information and the real-time ultrasonic imaging information on the same display interface. The user can combine the real-time imaging information with the real-time ultrasonic imaging information to provide guidance to remote ultrasonic operators, observe the complexion of the patient, and conduct ultrasonic diagnosis.

In summary, the medical information display system of this disclosure can be configured to store medical information of the target object, which includes at least two types of vital sign information, examination information, treatment information, and imaging information of the target object, and can also display detailed information of the selected medical information of the target object on the display device, which makes it more convenient for the user to view and analyze at least two types of medical information of the selected target object, improving viewing and analysis efficiency.

Furthermore, this application also provides a medical system, which can include the aforementioned medical information display system; and at least two of the monitoring device, examination device, treatment device, and camera device, wherein the monitoring device is configured to acquire the vital sign information of the target object which is monitored by the monitoring device, the examination device is configured to exam the target object and generate the examination information of the target object, the treatment device is configured to treat the target object and output the treatment information, and the camera device is configured to acquire the imaging information of the target object.

The medical system of this application has the same advantages as the aforementioned medical information display system, as it has the aforementioned medical information display system.

Although exemplary embodiments have been described here with reference to the accompanying drawings, it should be understood that the aforementioned exemplary embodiments are only exemplary and are not intended to limit the scope of this disclosure to this extent. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps of each example described in combination with the disclosed embodiments in this disclosure can be implemented in electronic hardware, or a combination of computer software and electronic hardware. Whether these functions are executed in hardware or software depends on the specific application and design constraints of the technical solution. Professional technicians may use different methods to achieve the described functions for each specific application, but such implementation should not be considered beyond the scope of this disclosure.

In the several embodiments provided in this application, it should be understood that the disclosed devices and methods can be implemented in other ways. For example, the device embodiments described above are only illustrative. For example, the division of units is only a logical functional division, and there may be other division methods in actual implementation. For example, a plurality of units or components can be combined or integrated into another device, or some features can be ignored or not executed.

The description provided here provides a large number of specific details. However, it can be understood that embodiments of this disclosure can be practiced without these specific details. In some examples, well-known methods, structures, and techniques are not shown in detail to avoid blurring the understanding of this specification.

Similarly, it should be understood that in order to streamline this disclosure and assist in understanding one or more of the various aspects of this disclosure, in the description of exemplary embodiments of this disclosure, the various features of this disclosure are sometimes grouped together into a single embodiment, diagram, or description thereof. However, the method of this disclosure should not be interpreted as reflecting the intention that the claimed protection of this disclosure requires more features than those explicitly recorded in each claim. More precisely, as reflected in the corresponding claims, the inventive point is that the corresponding technical problem can be solved with less than all features of a single disclosed embodiment. Therefore, the claims following the specific implementation method are explicitly incorporated into the specific implementation method, where each claim itself serves as a separate embodiment of this disclosure.

Technicians in this field can understand that, in addition to mutual exclusion between features, any combination can be configured to combine all features disclosed in this specification (including accompanying claims, abstracts, and drawings), as well as all processes or units of any method or device so disclosed. Unless otherwise explicitly stated, each feature disclosed in this specification (including accompanying claims, abstracts, and accompanying drawings) may be replaced by alternative features that provide the same, equivalent, or similar purpose.

In addition, those skilled in the art can understand that although some embodiments herein include certain features included rather than other features in other embodiments, the combination of features of different embodiments means that they fall into the scope of this disclosure and form different embodiments. For example, in the claims, any one of the claimed embodiments can be used in any combination.

The embodiments of various components of this disclosure can be implemented in hardware, software modules running on one or more processors, or a combination of them. Technicians in this field should understand that a microprocessor or digital signal processor (DSP) can be used in practice to implement some or all of the functions of some modules according to embodiments of this disclosure. This disclosure can also be implemented as a device program (such as a computer program and a computer program product) for executing some or all of the methods described herein. The program implementing this disclosure can be stored on a computer-readable medium or can take the form of one or more signals. Such signals can be downloaded from internet websites, provided on carrier signals, or in any other form.

It should be noted that the above embodiments illustrate this disclosure rather than limit it, and those skilled in the art can design alternative embodiments without departing from the scope of the attached claims. In the claims, any reference symbol between parentheses should not be constructed as a restriction on the claims. This disclosure can be implemented with the help of hardware including several different components and with the help of appropriately programmed computers. Among the unit claims that list several devices, several of these devices can be specifically embodied through the same hardware item. The use of words first, second, and third does not indicate any order. These words can be interpreted as names.

## Claims

1. A medical information display system, **characterized in that**, comprising:
a communication interface, which is configured to communicatively connect with at least one monitoring device and/or at least one ultrasonic device, wherein each monitoring device is configured to acquire vital sign information of a target object which is monitored by the monitoring device, and each ultrasonic device is configured to perform an ultrasonic examination on a target object and generate ultrasonic information of said target object;
a storage device, which is configured to store executable program instructions, as well as to store the vital sign information which is acquired by the monitoring device and the ultrasonic information which is generated by the ultrasonic device;
a display device, which is configured to display the vital sign information and the ultrasonic information of one or more target objects;
one or more processors, which are configured to execute the program instructions which are stored in the storage device to enable the processor(s) to perform following steps:
obtaining the vital sign information which is acquired by the monitoring device and the ultrasonic information which is generated by the ultrasonic device through the communication interface;
controlling the storage device to store the vital sign information and the ultrasonic information according to a timestamp;
controlling the display device to provide a display observation interface, which comprises a plurality of display areas, wherein each display area correspondingly displays at least part of the vital sign information of one target object;
when obtaining a selection instruction for one display area of the plurality of display areas, controlling the display device to display a detail interface of an interested target object, which corresponds to the selected display area, wherein the detail interface is set with a review hotkey; and
when obtaining a review instruction for the review hotkey, controlling the display device to display a review interface for the interested target object on the detail interface, wherein the review interface is configured to display review information of the interested target object, and the review information comprises the vital sign information and the ultrasonic information of the interested target object; wherein, the ultrasonic information is displayed with the timestamp, or the vital sign information and the ultrasonic information are correlatively displayed based on the timestamp.

2. The medical information display system according to claim 1, **characterized in that**, the one or more processors are further configured to execute the program instructions which are stored in the storage device to enable the processor(s) to perform following additional step:
when obtaining a review instruction for the review information within a preset time period, controlling the display device to display, on the review interface, corresponding review information in a chronological order within the preset time period;
wherein the review interface displays a time marker which represents the chronological order, wherein an ultrasonic examination indication is provided at a time point of the time marker, which time point corresponds to each examination time of the ultrasonic information.

3. The medical information display system according to claim 2, **characterized in that**, the one or more processors are further configured to execute the program instructions which are stored in the storage device to enable the processor(s) to perform following additional step:
when obtaining a selection instruction for one ultrasonic examination indication, controlling the display device to display detailed information of the ultrasonic information which corresponds to the selected ultrasonic examination indication.

4. The medical information display system according to claim 2, **characterized in that**, the review information comprises an alarm event of the target object;
wherein the one or more processors are further configured to execute the program instructions which are stored in the storage device to enable the processor(s) to perform following additional step:
providing an alarm event indication at a time point of the time marker, which time point corresponds to the alarm event of the interested target object; wherein the time marker is provided with the ultrasonic examination indication; wherein the ultrasonic examination indication and the alarm event indication are displayed in different ways.

5. The medical information display system according to claim 1, **characterized in that**, the one or more processors are further configured to execute the program instructions which are stored in the storage device to enable the processor(s) to perform following additional step:
when obtaining a review instruction for the review information within a preset time period, controlling the display device to display, on the review interface, abbreviated information of the ultrasonic information within the preset time period in a list format;
wherein the abbreviated information of the ultrasonic information comprises an ultrasonic examination indication and time information of the ultrasonic examination.

6. The medical information display system according to claim 5, **characterized in that**, the one or more processors are further configured to execute the program instructions which are stored in the storage device to enable the processor(s) to perform following additional step:
when obtaining an ultrasonic detailed display instruction for one piece of the abbreviated information of the ultrasonic information which is displayed on the review interface, controlling the display device to display detailed information of the selected ultrasonic information on the review interface; or controlling the display device to display an ultrasonic window in a form of a floating window on a current interface of the detail interface, wherein the ultrasonic window displays detailed information of the ultrasonic information, and the detailed information of the ultrasonic information at least comprises an ultrasonic image.

7. The medical information display system according to claim 5, **characterized in that**, the one or more processors are further configured to execute the program instructions which are stored in the storage device to enable the processor(s) to perform following additional step:
controlling the display device to display, on the review interface, abbreviated information of an alarm event within the preset time period in a list format, wherein the abbreviated information of the alarm event and the abbreviated information of the ultrasonic information are arranged in a chronological order.

8. The medical information display system according to claim 1, **characterized in that**, the one or more processors are further configured to execute the program instructions which are stored in the storage device to enable the processor(s) to perform following additional steps:
providing an ultrasonic graph on the detail interface of the interested target object; and
when obtaining an input instruction for the ultrasonic graph, controlling the display device to display an ultrasonic window in a form of a floating window at a current interface of the detail interface, wherein the ultrasonic window displays the ultrasonic information.

9. The medical information display system according to any one of claims 2-8, **characterized in that**, the one or more processors are further configured to execute the program instructions which are stored in the storage device to enable the processor(s) to perform following additional step:
controlling the display device to display one or more pieces of the ultrasonic information of the same interested target object on the review interface or in an ultrasonic window.

10. The medical information display system according to claim 1, **characterized in that**, the detail interface of the interested target object is set with a video access hotkey;
wherein the one or more processors are further configured to execute the program instructions which are stored in the storage device to enable the processor(s) to perform following additional steps:
when displaying the review interface of the interested target object on the detail interface and obtaining a video viewing instruction for the video access hotkey; obtaining historical video information of the interested target object within a preset time period, and controlling the display device to display the historical video information of the interested target object.

11. The medical information display system according to claim 10, **characterized in that**, the historical video information and the ultrasonic information are correlatively displayed based on the timestamp; the historical video information is configured to provide examination operation information for the interested target object while acquiring the ultrasonic information.

12. The medical information display system according to claim 1, **characterized in that**, the detail interface of the interested target object is set with a video access hotkey;
wherein the one or more processors are further configured to execute the program instructions which are stored in the storage device to enable the processor(s) to perform following additional steps:
when displaying a real-time interface of the interested target object on the detail interface and obtaining a video viewing instruction for the video access hotkey; obtaining current real-time video information of the interested target object, and controlling the display device to display the current real-time video information of the interested target object.

13. The medical information display system according to claim 12, **characterized in that**, when the ultrasonic device exams the interested target object, the real-time video information is configured to display an examination process of the interested target object;
wherein the one or more processors are further configured to execute the program instructions which are stored in the storage device to enable the processor(s) to perform following additional steps:
obtaining examination operation guidance information which is required for the examination by the ultrasonic device, and transmitting the examination operation guidance information to the ultrasonic device through the communication interface.

14. The medical information display system according to any one of claims 10-13, **characterized in that**, the one or more processors are further configured to execute the program instructions which are stored in the storage device to enable the processor(s) to perform following additional step:
controlling to display at least one of followings on the video information in a form of a bullet-screen: an alarm event, the ultrasonic information, and at least part of the vital sign information.

15. The medical information display system according to claim 1, **characterized in that**:
each display area is set with a video access hotkey, wherein the one or more processors are further configured to execute the program instructions which are stored in the storage device to enable the processor(s) to perform following additional steps:
when obtaining a video viewing instruction for the video access hotkey, obtaining current real-time video information of the interested target object, which corresponds to the display area where the video access hotkey is located, and controlling the display device to display the real-time video information; or
the display observation interface is set with a video access hotkey, wherein the one or more processors are further configured to execute the program instructions which are stored in the storage device to enable the processor(s) to perform following additional steps:
when obtaining a video viewing instruction for the video access hotkey, obtaining current real-time video information of one or more target objects, and controlling the display device to simultaneously display the real-time video information of the one or more target objects.

16. The medical information display system according to claim 1, **characterized in that**:
when a plurality of monitoring devices exist, the communication interface communicatively connects with the plurality of monitoring devices; when a plurality of ultrasonic devices exist, the communication interface communicatively connects with the plurality of ultrasonic devices; or
when the communication interface communicatively connects with the monitoring device, the communication interface is further configured to receive the ultrasonic information of the ultrasonic device which communicatively connects with the monitoring device; or
when the communication interface communicatively connects with the ultrasonic device, the communication interface is further configured to receive the ultrasonic information of the ultrasonic device which is stored locally.

17. The medical information display system according to claim 1, **characterized in that**, the communication interface is further configured to transmit the vital sign information and/or the ultrasonic information which are/is stored by the storage device to the monitoring device, and to enable the monitoring device to display the vital sign information and/or the ultrasonic information.

18. The medical information display system according to any one of claims 1-17, **characterized in that**, the ultrasonic information comprises identification information of the interested target object and time information of the ultrasonic examination; wherein the ultrasonic information further comprises at least one of:
an ultrasonic image, an ultrasonic examination report, and a measurement result based on ultrasonic image(s);
the vital sign information comprises identification information of the interested target object, wherein the vital sign information further comprises vital sign parameter information of the target object, vital sign parameter wave(s) of the target object, and alarm event information of the target object.

19. The medical information display system according to claim 18, **characterized in that**, the identification information of the interested target object comprises a patient support number of the interested target object, name of the interested target object, identification code of the interested target object.

20. A medical information display system, **characterized in that**, comprising:
a communication interface, which is configured to communicatively connect with at least one of a monitoring device, an examination device, a treatment device, and a camera device, wherein the monitoring device is configured to acquire vital sign information of a target object which is monitored by the monitoring device, the examination device is configured to exam the target object and generate examination information of the target object, the treatment device is configured to treat the target object and output treatment information, the camera device is configured to acquire imaging information of the target object;
a storage device, which is configured to store executable program instructions, as well as to store medical information of the target object, wherein the medical information comprises at least two types of the vital sign information, the examination information, the treatment information, and the imaging information;
one or more processors, which are configured to execute the program instructions which are stored in the storage device to enable the processor(s) to perform following steps: obtaining the medical information of a plurality of target objects through the communication interface; wherein the medical information of each target object comprises at least two types of the vital sign information, the examination information, the treatment information, and the imaging information; controlling the storage device to store the obtained medical information; and controlling the display device to display the medical information of the plurality of target objects; and controlling, when a selection instruction for one target object is received, the display device to display detailed information of the medical information of a selected target object; and
a display device, which is configured to display the medical information.

21. The medical information display system according to claim 20, **characterized in that**, the one or more processors are further configured to execute the program instructions which are stored in the storage device to enable the processor(s) to perform following additional steps:
controlling the display device to provide a display observation interface, which comprises a plurality of display areas, wherein each display area correspondingly displays at least part of the medical information of one target object; and
when obtaining a selection instruction for one display area of the plurality of display areas, controlling the display device to display a detail interface of an interested target object, which corresponds to the selected display area, wherein the detail interface comprises a plurality of display interfaces and each display interface is configured to display one type of the medical information.

22. The medical information display system according to claim 20, **characterized in that**, the display device provides a display observation interface, which comprises a plurality of display areas, wherein each display area correspondingly displays at least part of the medical information of one target object; each display area further correspondingly displays an icon of a medical device which is associated with one target object.

23. The medical information display system according to claim 20, **characterized in that**, the one or more processors are further configured to execute the program instructions which are stored in the storage device to enable the processor(s) to perform following additional steps:
controlling the display device to provide a display observation interface, which comprises a plurality of display areas, wherein each display area correspondingly displays at least part of the medical information of one target object; and
when obtaining a selection instruction for one display area of the plurality of display areas to select a target object, controlling the display device to display a detail interface of an interested target object, which corresponds to the selected display area, wherein the detail interface comprises a plurality of display interfaces and each display interface is configured to display at least two types of the medical information.

24. The medical information display system according to claim 20, **characterized in that**, the one or more processors are further configured to execute the program instructions which are stored in the storage device to enable the processor(s) to perform following additional step:
storing the vital sign information, the examination information, the treatment information, and the imaging information according to a timestamp.

25. The medical information display system according to any one of claims 20-24, **characterized in that**, the vital sign information comprises at least one type of following information: vital sign parameter information of the target object, and alarm event information;
the examination information comprises at least one type of following information: medical imaging information, medical examination report, and medical diagnostic result;
the treatment information comprises at least one type of following information: administration information, ventilation parameter information, and defibrillation treatment parameter information;
the imaging information, which is acquired by the camera device, comprises at least one type of following information: video information of the target object, and image information of the target object;
the video information of the target object and/or the image information of the target object comprise/comprises at least one type of following information: body surface information of the target object, dynamic information of the target object, examination or treatment operation information performed on the target object.

26. The medical information display system according to any one of claims 20-25, **characterized in that**, when the examination device comprises an ultrasonic device, the one or more processors are further configured to execute the program instructions which are stored in the storage device to enable the processor(s) to perform following additional steps:
obtaining real-time imaging information of the target object which is acquired by the camera device, and real-time ultrasonic imaging information of the target object which is measured by the ultrasonic device; and
controlling the display device to display the real-time imaging information and the real-time ultrasonic imaging information at a same display interface.

27. The medical information display system according to any one of claims 20-26, **characterized in that**, the examination device comprises at least one type of an ultrasonic device and an X-ray imaging device;
the treatment device comprises at least one type of a ventilator, a defibrillator, an infusion pump, an oxygen therapy instrument, an anesthesia machine and an extracorporeal membrane oxygenation.

28. A medical system, **characterized in that**, comprising:
at least one monitoring device, wherein each monitoring device is configured to acquire vital sign information of a target object which is monitored by the monitoring device;
at least one ultrasonic device, wherein each ultrasonic device is configured to perform an ultrasonic examination on the target object and generate ultrasonic information of the target object; and
the medical information display system according to any one of claims 1-19.

29. A medical system, **characterized in that**, comprising:
the medical information display system according to any one of claims 20-27; and
at least two of the monitoring device, the examination device, the treatment device, and the camera device, wherein the monitoring device is configured to acquire the vital sign information of the target object which is monitored by the monitoring device, the examination device is configured to exam the target object and generate the examination information of the target object, and the treatment device is configured to treat the target object and output the treatment information, the camera device is configured to acquire the imaging information of the target object.
